# EUROPÄISCHE PATENTSCHRIFT

(11) **EP 0 588 782 B1**
(45) Veröffentlichungstag und Bekanntmachung des Hinweises auf die Patenterteilung: **05.11.1997**
(21) Anmeldenummer: 93890180.8
(22) Anmeldetag: 10.09.1993
(51) Int. Cl.: G01N 1/12, G01N 33/18, B65D 51/00

(54) **Probensammler**
Water sampler
Dispositif de prise d'échantillons

(30) Priorität: 14.09.1992 AT 1829/92
(43) Veröffentlichungstag der Anmeldung: 23.03.1994
(73) Patentinhaber: Niederreiter, Richard, 5310 Mondsee (AT)
(72) Erfinder: Niederreiter, Richard, 5310 Mondsee (AT)
(74) Vertreter: Itze, Peter, Dipl.-Ing.

(56) Entgegenhaltungen:
- WO-A-89/12220
- AU-B- 522 927
- DE-A- 2 803 593
- FR-A- 2 423 767
- US-A- 3 961 721

## Beschreibung

Probensammler zur Ziehung von Wasserproben aus Gewässern, Brunnen, Bohrlöchem und dergleichen

Die Erfindung betrifft einen Probensammler für Wasserproben aus tiefen Gewässern, Bohrlöchern, Brunnen und dergleichen, der es ermöglicht rasch Wasserproben aus beliebigen Tiefen zu ziehen, wobei eine Verzerrung des Analysenergebnisses durch auftretende Flüssi keitswirbel vermieden wird.

Moderne Wasselschöpfen (vgl. DE-A-2 803 593), welche z.B. innerhalb von Bohrlöchern für Trinkwasserbrunnen abgesenkt werden, bestehen im wesentlichen aus einem Hohlzylinder der an den beiden offenen Enden mit speziell ausgebildeten Verschlußeinrichtungen versehen ist. Solange der Wasserschöpfer abgesenkt wird, stehen beide Verschlüsse offen und das Wasser kann durch den Hohlzylinder fließen. Sobald die gewünschte Wassertiefe erreicht ist, werden beide Verschlüsse gleichzeitig geschlossen.

Die Verschlußeinrichtungen werden dabei entweder elektrisch, elektromagnetisch oder mechanisch über ein Gestänge oder ein absenkbares Fallgewicht, welches über Federwirkung den Verschluß auslöst, betätigt.

Die elektrische oder elektromagnetische Auslösung der Verschlußeinrichtungen hat den Nachteil, daß sie eine Stromversorgungsleitung zur Oberfläche benötigt, was bei größeren Probenahmetiefen zu Problemen führt. Wird die Stromversorgung über eine am Probensammlergehäuse angebrachte Stromquelle durchgeführt, so ist ein komplizierterer Aufbau mit einer isolierten Batteriekammer nötig.

Die mechanische Verschlußauslösung über ein Gestänge ist nur bei geringen Tiefen anwendbar. Wird der mechanische Verschluß durch ein am Halteseil abgesenktes Fallgewicht ausgelöst, so bedingt das bei größeren Tiefen einen erhöhten Zeitaufwand. Zudem wird die Zuverlässigkeit des Systems geringer.

Bei einer anderen Bauweise (AU-B-522 927) werden die Verschlußplatten beim Absenken durch den Wasserdruck in geöffneter Stellung gehalten. Sobald der Probensammler nicht mehr abgesenkt wird, schließen die Klappen aufgrund ihres eigenen Gewichtes. Beim Heben des Probensammlers wirkt der Wasserdruck auf die obere Klappe und hält diese geschlossen. Auf die untere Klappe wirkt jedoch kein Wasserdruck, weshalb diese beim Heben des Probensammlers zu "flattern" beginnt. Dadurch kommt es zu einer Vermischung des Sammlerinhaltes mit dem Wasser aus anderen Wasserschichten und damit zu einer Verzerrung des Analysenergebnisses. Außerdem ist dieses System ist für Probensammler mit rundem Querschnitt nicht brauchbar, da die untere Verschlußklappe in geöffneter Stellung schräg zur Durchflußrichtung des Wassers steht. Es entstehen hinter der Klappe starke Strömungswirbel, welche eine Vermischung des Wassers aus verschiedenen Tiefen und damit wiederum eine Verzerrung des Analysenergebnisses bewirken. Speziell bei schnellerem Absenken in offenen Gewässern führt die Schrägstellung der unteren Nappe dazu, daß der Probensammler seitlich ausweicht und somit schräg absinkt. Es ist dann nicht mehr möglich, die Tiefe der Wasserprobe exakt zu bestimmen. Um das zu vermeiden, müßte der Probensammler sehr langsam abgesenkt werden, was wiederum einen wesentlich erhöhten Zeitaufwand erfordert.

Die Erfindung stellt sich daher die Aufgabe, eine Verschlußeinrichtung für solche Probensammler zu schaffen, welche die Nachteile herkömmlicher Verschlüsse vermeidet.

Diese Aufgabe wird erfindungsgemäß dadurch gelöst, daß die untere Verschlußklappe aus dauerelastischem Material besteht, in welches mehrere Metallteile eingelagert sind. Dadurch ist es ermöglicht, daß die Verschlußklappe der Strömung des eintretenden Wassers so ausweichen kann, daß eine Wirbelbildung ausgeschlossen ist.

Vorteilhafterweise können bei einem mit kreisrundem Verschlußklappen abgeschlossenen Rohr die Metallteile aus mindestens einem mittig liegenden Metallstreifen und zwei randliegenden kreissegmentförmigen Metallstreifen bestehen, die senkrecht zur Achse der Dreheinrichtung der Verschlußklappe ausgerichtet sind, wobei die zwischen den Metallteilen freien Bereiche der Verschlußklappe flexibel sind. Auf diese Art ist es auch möglich, Probensammler kreisrunden Querschnitts zu verwenden, wobei sich die Verschlußklappen aufgrund der flexiblen Bereiche an die Innenwandung des Rohres anlegen, wobei auf diese Weise ein wirbelfreies Einströmen der Flüssigkeit in die Probesammlerkammer erfolgen kann. Weiters kann die obere Verschlußklappe über eine Schnur mit der unteren Verschlußklappe verbunden sein, wobei die Schnurlänge so gewählt ist, daß die untere Verschlußklappe bei in Offenstellung gehaltener oberer Klappe und bei gespannter Schnur durch diese in leicht schräger geöffneter Stellung gehalten wird. Beim Absenken des Probesammlers in das Wasser wird die untere Verschlußklappe unter Anschmiegen an die Innenwandung des Rohres völlig geöffnet, wodurch ein wirbelfreies Einströmen der Flüssigkeit in den Probensammler ermöglicht ist. Bei Beendigung der Absenkungsbewegung nimmt die untere Verschlußklappe wieder die leicht schräge, geöffnete Stellung ein, sodaß bei ruckartigem Hochziehen des Probesammlers ein Staudruck an der Oberseite der Verschlußklappe entsteht, welcher über die Schnur ein Lösen der oberen Verschlußklappe von ihrer in Offenstellung gehaltener Lage erreicht wird, sodaß sowohl die untere als auch die obere Verschlußklappe in ihre Schließstellung gelangen können. In vorteilhafter Weise kann über dem mittleren Metallstreifen der unteren Verschlußklappe am dauerelastischen Material eine Öse zum Befestigen der Schnur vorgesehen sein und das dauerelastische Material am äußeren Rand der Klappe in eine Dichtungslippe auslaufen, wodurch eine einfache Anbringungsmöglichkeit der Schnur und ein gutes selbsttätiges Abdichten der Verschlußklappe erzielt ist. Für ein sicheres Festhalten der unteren Verschlußklappe kann diese in geschlossener Stellung über mehrere ringförmig angeordnete Magnete, welche in einem schweren Metallring eingelassen sind, in dicht geschlossener Stellung gehalten sein, wobei dieser Metallring den unteren Abschluß des Probensammelrohres bildet. Dies ergibt einerseits ein gutes Abdichten der Verschlußklappe, da die Magnete über die Metallstreifen die untere Verschlußklappe so auf den schweren Metallring ziehen, daß über die Dichtungslippe ein dichter Abschluß gegeben ist, wobei der schwere Metallring zusätzlich noch die Möglichkeit bietet, daß ein rasches Absenken des Probesammlers in die Flüssigkeit erzielbar ist.

Weiters kann die obere Verschlußklappe in geschlossener Stellung auf einem leichten Metallring aufliegen, wobei dieser Metallring den oberen Abschluß des Probensammelrohres bildet und mit einem Aufhängebügel, an dem der Probesammler aufgehängt ist, lösbar verbunden ist. Damit wird einerseits erzielt, daß aufgrund der Gewichtsdifferenz der Metallringe das untere Ende immer zuverlässig nach unten gezogen wird, wobei anderseits die obere Verschlußklappe sich ungehindert öffnen kann, um so beim Einsenken des Probensammelrohres in die Flüssigkeit das Einströmen dieser Flüssigkeit in den Probensammler nicht zu behindern.

Schließlich kann die obere Verschlußklappe in geöffneter Stellung von einem Magneten festhaltbar sein, der im Aufhängebügel eingelassen ist. Auf diese Art kann über die Wahl des Magneten die Haltekraft vorgegeben werden, wobei diese Haltekraft nicht zu groß sein darf, um ein leichtes Schließen durch ein ruckartiges Anheben des Probesammlers zu erreichen.

In der Zeichnung ist ein Ausführungsbeispiel des Erfindungsgegenstandes dargestellt.

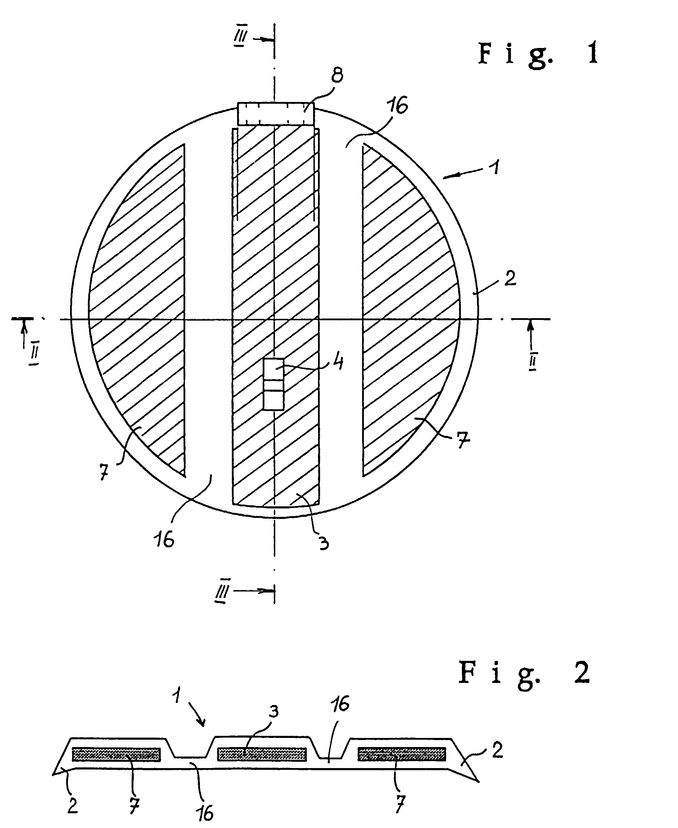

Fig. 1 zeigt eine Draufsicht auf die untere Verschlußklappe.

Fig. 2 ist ein Schnitt nach Linie II-II der Fig. 1.

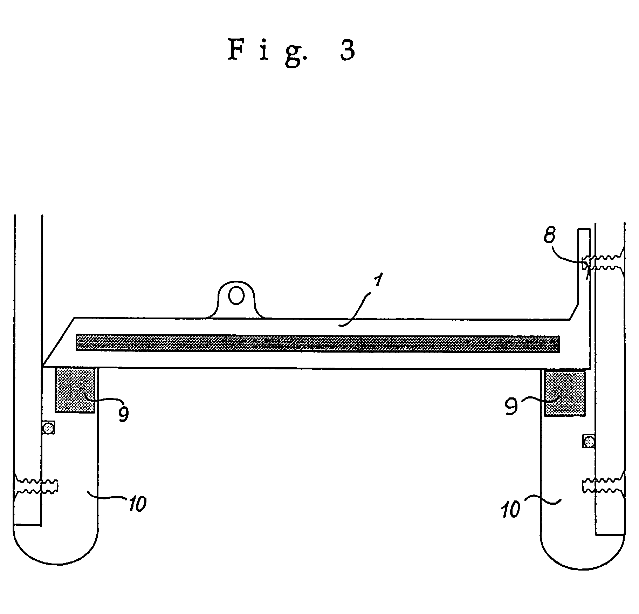

Fig. 3 gibt einen Schnitt nach Linie III-III der Fig. 1 wieder, u.zw. mit dem angrenzenden unteren Bereich des Rohres.

Fig. 4 zeigt schematisch den Probensammler im Vertikalschnitt in Ausgangsstellung vor dem Absenken in die Flüssigkeit.

Fig. 5 gibt den Probensammler während des Einsenkens in die Flüssigkeit wieder.

Mit 1 ist die untere Verschlußklappe bezeichnet, welche aus flexiblem, dauerelastischem Material, wie z.B. aus Silikongummi besteht, wobei das dauerelastische Material randseitig der Verschlußklappe 1 in eine Dichtungslippe 2 ausläuft. In das dauerelastische Material sind mehrere Metallteile eingelassen, wobei ein quer durch den mittleren Bereich der Verschlußklappe verlaufender Metallstreifen 3 vorgesehen ist, der mit seinem einen Ende von einer Dreheinrichtung 8, mit welcher die Verschlußklappe 1 mit dem die Probensammeleinrichtung bildenden Rohr 11 verbunden ist. Neben dem Metallstreifen 3 sind noch je ein äußerer, kreissegmentförmiger Metallteil 7 vorgesehen, wobei zwischen dem Metallstreifen 3 und den seitlich davon befindlichen Metallteilen 7 flexible Bereiche 16 vorgesehen sind, welche quer zur Achse der Dreheinrichtung verlaufen und damit ein Abknicken der Verschlußklappe 1 um diese flexiblen Bereiche ermöglicht. Dies ergibt bei in das Innere des kreisrunden Rohres 11 geklappter Verschlußklappe 1, welche kreisrundförmigen Grundriß aufweist, ein Anlegen der Verschlußklappe an die Innenwandung des Rohres 11, wobei der Metallstreifen 3 und die flexiblen Bereiche 16 im wesentlichen in Richtung der Einströmung der Flüssigkeit verlaufen.

Das Probensammelrohr 11 ist dabei aus durchsichtigem Kunststoff, z.B. transparentem Acrylglas, gebildet, um dadurch eine visuelle Beurteilung der Wasserprobe zu ermöglichen.

Auf der in das Innere des Probensammelrohres 11 weisenden Seite der Verschlußklappe 1 ist eine Öse 4 vorgesehen, welche zur Verbindung mit einer Schnur 5 bestimmt ist, die die untere Verschlußklappe 1 mit einer oberen Verschlußklappe 6 verbindet. Diese obere Verschlußklappe 6 ist am oberen Ende des Probensammelrohres 11 schwenkbar gelagert, u.zw. im Bereich eines leichten Metallringes 15, welcher gleichzeitig als Auflage für die obere Verschlußklappe 6 dient. Die Schnur 5 ist an der oberen Verschlußklappe 6 über einen in Richtung zum Probensammelrohr 11 hin weisenden Arm 17 befestigt, welcher im wesentlichen im rechten Winkel von der Unterseite der oberen Verschlußklappe 6 wegragt. Dieser Arm 17 ist dabei so bemessen, daß sein freies Ende bei nach oben geklappter oberer Verschlußklappe 6 etwa in der Längsachse des Probensammelrohres 11 liegt.

An der Unterseite des Probensammelrohres 11 ist ein schwerer Metallring 10 vorgesehen, in welchem Magnete 9 eingelassen sind. Diese Magnete dienen dazu, die Metalleinlagen 3 und 7 nach unten zu ziehen, um dann über die Dichtlippen 2 ein dichtes Abschließen der unteren Verschlußklappe mit dem schweren Metallring einerseits und auch mit der Innenwandung des Probenrohres 11 zu erreichen.

An der Oberseite des Probesammelrohres 11 ist ein Aufhängebügel 12 vorgesehen, dessen einer Schenkel im Bereich der Anlenkung der oberen Verschlußklappe 6 am Probensammelrohr 11 angreift, und der andere Schwenkel etwa im gegenüberliegenden Bereich des Probesammelrohres. Der im Bereich der Anlenkung der oberen Verschlußklappe 6 angreifende Schenkel des Aufhängebügels 12 ist mit einem Magnethalter 13 versehen, welcher dazu dient, die obere Verschlußklappe 6 in Offenstellung zu halten.

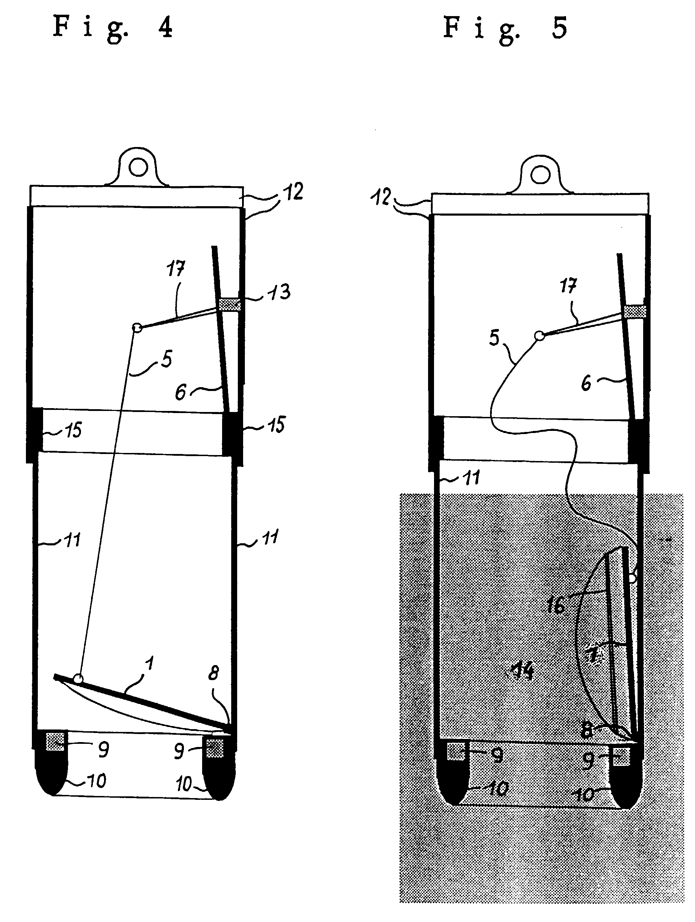

Mit 14 ist in Fig. 5 die Flüssigkeit angedeutet, aus welcher eine Probe gezogen werden soll.

Soll mittels des erfindungsgemäßen Probesammlers eine Probe aus der Flüssigkeit 14 gezogen werden, dann wird die obere Verschlußklappe in Anlage an die Magnethalterung 13 gebracht, wobei über den Arm 17 und die Schnur 5 die untere Verschlußklappe 1 in der leicht schräg nach oben weisenden Stellung gehalten wird, wie dies in Fig. 4 gezeigt ist. Beim Einsenken des Probensammlers in die Flüssigkeit 14 wird durch den Strömungswiderstand die untere Verschlußklappe 1 in das Innere des Probensammelrohres 11 verschwenkt, und nimmt die in Fig. 5 wiedergegebene Stellung ein. Beide Verschlußklappen befinden sich damit in völlig geöffneter Stellung.

Sobald die Absenkbewegung beendet ist, senkt sich die untere Verschlußklappe 1 aufgrund ihres Gewichtes wieder in die in Fig. 4 wiedergegebene Stellung, in welcher sie nach wie vor über die Magnethalterung 13, den Arm 17 und die Schnur 5 gehalten ist. Soll nun der Probesammler abgeschlossen werden, dann wird über den Aufhängebügel 12 eine ruckartige Bewegung im Sinne eines Hochziehens des Probesammlers ausgeführt, wodurch am oberen Teil der unteren Verschlußklappe 1 ein Staudruck entsteht, der zu einem Spannen der Schnur 5 und in weiterer Folge zu einem Lösen der oberen Verschlußklappe 6 von dem Magnethalter 13 führt. Aufgrund des Eigengewichtes und des entstandenen Flüssigkeitsstaus werden nun beide Verschlußklappen geschlossen, wobei die untere Verschlußklappe 1 am Metallring 10 und die obere Verschlußklappe 6 am Metallring 11 anliegt. Die untere Verschlußklappe ist dabei aufgrund der Wirkung der Magnete 9 auf die Metallstreifen 3 und 7 und auch aufgrund der Wirkung der Dichtlippe so dicht, daß ein Ausfließen der im Probensammler 11 befindlichen Flüssigkeit verhindert ist. Durch die obere Verschlußklappe 6, welche sich ebenfalls in Schließstellung befindet, wird ein nachträgliches Vermischen der aus größeren Tiefen entnommenen Flüssigkeit mit Flüssigkeit geringer Tiefe vermieden.

Der erfindungsgemäße Probensammler ist somit sowohl hinsichtlich des Aufbaues als auch hinsichtlich der Handhabung sehr einfach und benötigt sehr wenig Raum innerhalb eines Rohres oder eines Brunnens, da die Auslösung des Schließens des Probesammlers einzig und allein mit Hilfe der Aufhängeschnur vorgenommen wird, indem ein ruckartiges Hochziehen des Probesammelrohres vorgenommen wird, was dann das dichte Abschließen des Probesammelrohres 11 bewirkt.

Nach Hochziehen des Probesammlers kann dann die Flüssigkeit in bereitgestellte Flaschen u.dgl. abgefüllt werden, wobei nach Festlegen der oberen Verschlußklappe 6 am Haltemagnet 13 der Probesammler bereits für eine nächste Probenziehung vorbereitet ist.

## Patentansprüche

1. Probensammler für Wasserproben aus tiefen Gewässern, Bohrlöchern, Brunnen und dergleichen, welcher aus einem vorzugsweise durchsichtigen Rohr (11) besteht, das an den beiden Enden mit Verschlußklappen (1,6) verschlossen wird, dadurch gekennzeichnet, daß die untere Verschlußklappe (1) aus dauerelastischem Material besteht, in welches mehrere Metallteile (3,7) eingelagert sind.

2. Probensammler nach Anspruch 1, dadurch gekennzeichnet, daß bei einem mit kreisrunden Verschlußklappen (1,6) abgeschlossenen Rohr die Metallteile aus mindestens einem mittig liegenden Metallstreifen (3) und zwei randliegenden kreissegmentförmigen Metallteilen (7) bestehen, die senkrecht zur Achse der Dreheinrichtung (8) der Verschlußklappe (1) ausgerichtet sind, wobei die zwischen den Metallteilen (3,7) freien Bereiche (16) der Verschlußklappe (1) flexibel sind.

3. Probensammler nach Anspruch 1 oder 2, dadurch gekennzeichnet, daß die obere Verschlußklappe (6) über eine Schnur (5) mit der unteren Verschlußklappe (1) verbunden ist, wobei die Schnurlänge so gewählt ist, daß die untere Verschlußklappe (1) bei in Offenstellung gehaltener oberer Klappe (6) und bei gespannter Schnur durch diese in leicht schräger, geöffneter Stellung gehalten wird.

4. Probensammler nach Anspruch 1, 2 oder 3, dadurch gekennzeichnet, daß über dem mittleren Metallstreifen (3) der unteren Verschlußklappe (1) am dauerelastischen Material eine Öse (4) zum Befestigen der Schnur (5) vorgesehen ist und das dauerelastische Material am äußeren Rand der Klappe in einer Dichtungslippe (2) ausläuft.

5. Probensammler nach einem der Ansprüche 1 bis 4, dadurch gekennzeichnet, daß die untere Verschlußklappe (1) in geschlossener Stellung über mehrere, ringförmig angeordnete Magnete (9), welche in einem schweren Metallring (10) eingelassen sind, in dicht geschlossener Stellung gehalten wird, wobei dieser Metallring den unteren Abschluß des Probensammlerrohres (11) bildet.

6. Probensammler nach einem der Ansprüche 1 bis 5, dadurch gekennzeichnet, daß die obere Verschlußklappe (6) in geschlossener Stellung auf einem leichten Metallring (15) aufliegt, wobei dieser Metallring den oberen Abschluß des Probensammlerrohres (11) bildet und mit einem Aufhängebügel (12), an dem der Probensammler aufgehängt ist, lösbar verbunden ist.

7. Probensammler nach einem der Ansprüche 1 bis 6, dadurch gekennzeichnet, daß die obere Verschlußklappe (6) in geöffneter Stellung von einem Magneten (13) festhaltbar ist, der im Aufhängebügel (12) eingelassen ist.

## Claims

1. A sampler for water samples from deep waters, boreholes, wells and the like, which consists of a preferably transparent tube (11) which is sealed at either end with sealing flaps (1, 6), characterized in that the lower sealing flap (1) is made of permanently elastic material in which several metal elements (3, 7) are included.

2. A sampler as claimed in claim 1, characterized in that in a tube which is sealed with circular sealing flaps (1, 6) the metal elements consist of at ) least one centrally disposed metal strip (3) and two circular-segment-like metal elements (7) which are situated at the edge and are aligned perpendicular to the axis of the rotating device (8) of the sealing flap (1), with the free areas (16) of the sealing flap (1) which are between the metal elements (3, 7) being flexible.

3. A sampler as claimed in claim 1 or 2, characterized in that the upper sealing flap (6) is connected with the lower sealing flap (1) by way of a cord (5), with the length of the string being selected in such a way that the lower sealing flap (1), when the upper flap (6) is held in the opened position and when the string is tensioned, is held in a slightly inclined opened position by the same.

4. A sampler as claimed in claim 1, 2 or 3, characterized in that an eye (4) for attaching the cord (5) is provided above the central metal strip (3) of the lower sealing flap (1) on the permanently elastic material and that the permanently elastic material ends in a sealing lip (2) on the outer edge of the flap.

5. A sampler as claimed in one of the claims 1 to 4, characterized in that in the closed position the lower sealing flap (1) is held in a tightly closed position by way of several, annularly arranged magnets (9) which are embedded in a heavy metal ring (10), with this metal ring forming the lower end of the sampler tube (11).

6. A sampler as claimed in one of the claims 1 to 5, characterized in that the upper sealing flap (6) rests in the closed position on a light metal ring (15), with this metal ring forming the upper end of the sampler tube (11) and being detachably connected with a suspension bracket (12) on which the sampler is suspended.

7. A sampler as claimed in one of the claims 1 to 6, characterized in that the upper sealing flap (6) can be held in the opened position by a magnet (13) which is embedded in the suspension bracket (12).

## Revendications

1. Dispositif de prise d'échantillon pour des échantillons d'eau provenant d'eaux profondes, de trous de sondage, de puits et autres, lequel se compose d'un tube (11) de préférence transparent dont chaque extrémité est fermée par des clapets (1,6), caractérisé en ce que le clapet inférieur est réalisé dans un matériau à élasticité permanente dans lequel on a inséré plusieurs parties métalliques (3,7).

2. Dispositif de prise d'échantillon selon la revendication 1 caractérisé en ce que sur un tube fermé par des clapets (1,6) circulaires, les parties métalliques comprennent au moins un bande métallique (3) placée au centre et deux parties métalliques (7) en forme de segment de cercle situées sur les bords, lesquelles sont alignées verticalement par rapport à l'axe du dispositif de rotation (8) du clapet (1), les zones libres (16) du clapet (1) situées entre les parties métalliques (3,7) étant souples.

3. Dispositif de prise d'échantillon selon la revendication 1 ou 2 caractérisé en ce que le clapet supérieur (6) est relié au clapet inférieur (1) par un cordon (5) dont la longueur a été sélectionnée de manière à ce que le clapet inférieur (1) soit maintenu par le cordon dans une position ouverte, légèrement inclinée lorsque le clapet supérieur (6) est tenu en position ouverte et que le cordon est tendu.

4. Dispositif de prise d'échantillon selon la revendication 1, 2 ou 3 caractérisé en ce que, pour attacher le cordon (5), on a prévu un il (4) sur le matériau à élasticité permanente au-dessus de la bande métallique (3) médiane du clapet inférieur (1) et en ce que le matériau à élasticité permanente se termine par une lèvre d'étanchéité (2) à la hauteur du bord extérieur du clapet.

5. Dispositif de prise d'échantillon selon l'une des revendications 1 à 4 caractérisé en ce qu'en position fermée, le clapet inférieur (1) est maintenu étanchement fermé au moyen de plusieurs aimants (9) disposés de façon annulaire, lesquels sont emboîtés dans une bague métallique (10) lourde formant la fermeture inférieure du tube (11) du dispositif de prise d'échantillon.

6. Dispositif de prise d'échantillon selon l'une des revendications 1 à 5 caractérisé en ce qu'en position fermée, le clapet supérieur (6) repose sur une bague métallique (15) légère formant la fermeture supérieure du tube (11) du dispositif de prise d'échantillon et est relié de façon détachable à un arceau de suspension (12) auquel le dispositif de prise d'échantillon est suspendu.

7. Dispositif de prise d'échantillon selon l'une des revendications 1 à 6 caractérisé en ce que le clapet supérieur (6) peut être maintenu en position ouverte par un aimant (13) qui est emboîté dans l'arceau de suspension (12).
